# EUROPEAN PATENT APPLICATION

(11) **EP 2 486 895 A2**
(43) Date of publication of application: **15.08.2012**
(21) Application number: 11188570.3
(22) Date of filing: 10.11.2011
(51) Int. Cl.: A61F 2/30, A61F 2/50, G06F 19/00, A61B 19/00, A61B 17/00, A61B 17/56

(54) **Additive manufacturing flow for the production of patient-specific devices comprising unique patient-specific identifiers**

(30) Priority: 10.11.2010 BE 201000668
(71) Applicant: Materialise NV, 3001 Leuven (BE)
(72) Inventor: Vancraen, Wilfried, 3040 Huldenberg (BE); Janssens, Michel, 1390 Grez-Doiceau (BE)
(74) Representative: Paemen, Liesbet R.J.

(57) **Abstract**

The invention relates to improved methods for the production of patient-specific medical devices such as patient-specific (surgical) guides, orthoses and prostheses based on unique patient-specific identifiers, where the manufactured patient-specific medical device is rejected or accepted on the basis of a comparison between geometrical dimensions of the device and the same geometrical dimensions in patient-specific images.

## Description

### TECHNICAL FIELD

The present invention relates to patient-specific medical devices, more specifically to patient-specific (surgical) guides, orthoses and prostheses, for instance patient-specific implants. More in particular, the present invention relates to methods for the production of patient-specific medical devices such as patient-specific (surgical) guides, orthoses and prostheses and patient-specific surgical guides, orthoses and prostheses obtained through these methods.

### STATE OF THE ART

Since the end of the previous century, technology has been developed to enable the use of three-dimensional images of a patient's pathology on the basis of Computed Tomography (CT) or Magnetic Resonance (MR) for the production of patient-specific implants and in addition, for support of several types of surgical procedures through patient-specific drilling templates. This allows for perfectly tailoring the design of the medical device as well as its attachment to bone and tissue to the patient's anatomy and the operating framework. This is described, for instance, in European patent no. 0 756 735.

In view of the increasing success of this technology that is currently being applied to thousands of patients around the world, it is of crucial importance for the industry to be able to guarantee that the production of these medical devices is failsafe. Although in the beginning, surgeons would systematically check implants or templates in the course of the operation, today they almost blindly rely on failsafe production. This implies that the manufacturer must be able to guarantee that within permitted tolerances, the patient-specific tool corresponds with the patient-specific anatomy and/or the implementation of the surgical plan.

Absence of guaranteed accuracy of the medical tool strongly reduces the added value of the patient-specificity or of the device itself (e.g. guides). For most experienced surgeons, a template is an instrument for preventing inadmissible deviations.

In order to ensure the accuracy of patient-specific medical devices, efforts are made to limit variations in each step of the design and manufacturing process to a minimum. Specially appointed quality inspectors check each individual design during the design phase. Very often there are procedures and checklists indicating which points and methods must be included in the checks.

Practically all available traditional measuring instruments, ranging from simple approval and rejection calibrators to the more complex 'Coordinate Measurement Machines' and 'Optical Scanners' are being used to check the patient-specific medical devices for accuracy. This always involves a check of the dimensions of a certain feature or even of the entire implant or template to verify deviations from the design.

However, designers are not infallible and in addition, equipment such as production machines may become disrupted, resulting in errors during the manufacture of the medical device. The only way to intercept all errors is to carry out a check during or after each design phase or production step. However, many of these checks are performed manually and therefore are not necessarily consistent and 100% reliable. Furthermore, the sequence of the production steps may generate cumulative errors that fall outside the tolerance limits.

Because each patient-specific medical device is tailored to a specific patient, ruling out serial production, it is difficult to perform the random checks often performed in other manufacturing processes to reduce the costs of control. Checking each step hence considerably adds to the price of the end product.

There is a need for improved or optimised methods of production of patient-specific medical devices such as implants and surgical models; methods that will create increased reliability of the devices for the patient en that can be implemented more cost-efficiently than the methods that involve a check at each step.

### SUMMARY OF THE INVENTION

The present invention overcomes one or more of the above-mentioned disadvantages of known methods for the production of patient-specific surgical medical devices by providing optimised production methods that are more time- and cost-efficient and which check, in one single step, the critical functionality of the implant or template by making a direct comparison between the end product and the data (derived) from the original patient-specific preoperative planning. In addition, this process is enhanced by a unique identification process that allows for flawless connection of any patient-specific medical device to the relevant patient.

According to a first aspect, the present invention provides optimised production methods for medical devices; characteristic of these methods is that the values of one or more critical dimensions for one or more functional elements of the produced medical devices are being compared to the values of these critical dimensions as determined by the planning. More specifically, the invention provides methods for producing patient-specific surgical guides and implants, which methods feature at least the following steps:
- a comparison between the values of one or more critical dimensions for one or more functional elements as derived directly from the geometry of the patient-specific surgical guide or implant and the values of these critical dimensions as derived directly from the data of the preoperative planning, and
- the approval or disapproval of the patient-specific surgical guide or implant on the basis of the comparison carried out in step a).

Typically, the methods of the present invention thus comprise the steps of
1) collecting three-dimensional patient-specific images;
2) planning the surgical procedure based on said three-dimensional patient-specific images and the patient-specific values derived therefrom;
3) designing the medical device based on the planning data, the patient-specific images and the patient-specific values derived therefrom; and
4) creating the medical device based on the design, and
5) determining the suitability of the patient-specific medical device based a comparison between the values of one or more critical dimensions for one or more functional elements as derived directly from the geometry of the patient-specific surgical guide or implant and the values of these critical dimensions as derived directly from the data of the preoperative planning, and approving or disapproving the patient-specific surgical guide or implant based thereon.

Production methods for patient-specific medical devices are usually characterised by the fact that the patient-specific devices, such as templates or implants, are created from a design which in itself is based on the planning data of the surgical procedure, the patient-specific images and the patient-specific values derived thereof.

The production of patient-specific medical devices thus usually comprises the following steps: collecting three-dimensional patient-specific images; planning the surgical procedure and the design of the medical device, based on the three-dimensional patient-specific images and the patient-specific values derived thereof; designing the medical device based on the planning data, the patient-specific images and the patient-specific values derived thereof; and finally, creating the medical device based on the design. In some cases, planning and design will run simultaneously. In addition, the methods of the present invention are characterised by the fact that the values of one or more critical dimensions for one or more functional elements of the (either or not completely) produced medical device are compared with the values of one or more corresponding critical dimensions that, based on the planning data, were defined for one or more of the above-mentioned functional elements.

In certain embodiments of the production methods according to the invention, the step of comparing the values of one or more critical dimensions for one or more functional elements is carried out at the end of the production process. Additionally or alternatively, the comparison may be carried out following one or more intermediate steps of the production process.

In certain embodiments of the production methods according to the invention, the step of comparing the values of one or more critical dimensions for one or more functional elements comprises the following intermediate steps:
i) identifying one or more critical dimensions for one or more functional elements of the produced patient-specific medical device
ii) defining the values of one or more critical dimensions identified in step i), by directly deriving these values from the preoperative planning data
iii) defining the values of one or more critical dimensions identified in step i), by directly deriving these values from the geometry of the produced patient-specific medical device, and
iv) comparing the values of one or more critical dimensions, defined in step ii), with the values of one or more critical dimensions, defined in step iii).

In certain specific embodiments of the production methods according to the present invention, defining the values of one or more critical dimensions of the (either or not completely) produced patient-specific medical device will be done via measurement, e.g. optical measurement of the geometry of the medical device.

In certain embodiments of the production methods according to the invention, defining the values of one or more critical dimensions for one or more functional elements of the patient-specific device will be done prior to disinfecting the produced object.

In certain embodiments of the production methods according to the invention, the step of comparing the values of one or more critical dimensions for one or more functional elements is preceded by establishing a unique link between the produced medical device and the patient and/or the original patient-specific images. There are several ways to realise this.

In certain embodiments, the produced medical device contains or will be equipped with a critical reference that will serve to realise the unique link between the produced patient-specific medical device and the patient and/or the original patient-specific images.

In certain embodiments, the critical reference will have the form of an identification code. In these embodiments, the identification code can, for instance, be integrated into the surface of the medical device in a three-dimensional format.

The methods of the present invention may comprise further steps in which, based on the comparison made, the patient-specific device is evaluated. In particular embodiments, the patient-specific device is discarded if based on the comparison described herein above, the device does not meet the required standards.

On the other hand, the link between the produced device and the patient and/or the images of the patient can also be realised based on inherent features of the device, such as the topology of the three-dimensional surface of the medical device.

In this context, the present invention developed a method to ensure a unique link between a produced patient-specific medical device and the original (segmented) patient images. In the context of the production methods of the present invention, the unique identification of the produced patient-specific medical device is being established via a unique link between the original patient-specific images and the produced medical device. More specifically, a statistical method is being used, such as Principal Component Analysis. This method, which applies to the identification of each patient-specific device, constitutes another aspect of the present invention.

According to a further aspect, the present invention provides methods for unique identification of patient-specific medical devices such as patient-specific surgical guides or templates, orthoses or prostheses with a patient. More specifically, these identification methods establish a unique link between the data of the produced patient-specific medical device and the original patient-specific images by using a statistical method, such as Principal Component Analysis (PCA).

In certain embodiments, the present invention provides methods for unique identification of a produced patient-specific medical device with the patient, establishing a unique link between the produced guide or the produced patient-specific implant and the original patient-specific images by using a method that assigns a unique combination of parameters to the geometry of the medical device. In certain embodiments, the unique identification methods of the present invention comprise the following steps:
(i) providing a set of reference geometries
(ii) calculating an average reference geometry based on the set of reference geometries
(iii) analysing the variation of the geometry of the medical device as compared to the average reference geometry, and
(iv) assigning a unique combination of parameters to the medical device that correspond with the most explicit variations as established in step (iii).

In certain embodiments of the identification methods according to the invention, the unique combination of parameters may be presented as a vector.

### FIGURES

The description below is illustrated with the following figures that should not be considered as limiting for the scope of the invention.
**Figure 1** provides an overview of a specific embodiment of the optimised production methods according to the invention.
**Figure 2** gives a detailed overview of a specific embodiment of the identification step of the production methods according to the present invention, using a statistical method to ensure a unique link between the guide or the implant and the original patient-specific images.
**Figure 3** shows how the preoperative step of the planning of a surgical procedure transpires according to the production methods of the present invention.
**Figure 4** gives a detailed overview of the different steps for the production of a patient-specific medical device according to certain embodiments of the present invention.
**Figure 5** shows the identification step according to certain embodiments of the production methods of the invention, whereby the unique link between the data of the medical device and the original patient-specific images is realised by using the geometry of the medical device.
**Figure 6** details the quality control step of the production methods according to certain embodiments of the present invention.
**Figure 7** shows certain embodiments of the production methods according to the invention in which the values of the critical dimensions have not been indicated directly in the planning but must first be derived or even calculated.
**Figure 8** shows a certain embodiment of the production methods according to the invention, in which, prior to the quality control step of the optical scan of the medical device, a 'pseudo-planning' is derived that is subsequently compared to the planning approved by the physician.
**Figure 9** shows certain embodiments of the production methods according to the invention, in which prior to the quality control step, so-called derived dimensions are calculated based on the values of the critical dimensions.
**Figures 10 and 11** reflect how the geometry of a certain guide or implant can be encrypted efficiently according to certain embodiments of the identification methods of the invention.
**Figure 12** illustrates how in certain embodiments of the production methods according to the invention, defining the values of one or more critical dimensions for one or more functional elements of the produced medical device takes place through measurement, whereby reference blocks (2) are attached to the medical device.
**Figure 13 and 14** illustrate a patient-specific medical device, more particularly a guide (3), which is positioned on a 1-angle table (not shown) in a certain position and a scanner which typically comprises a light emitting device which is positioned in between two cameras according to a particular embodiment of the invention.
**Figure 15** illustrates the intersection of the images taken by both cameras illustrated in

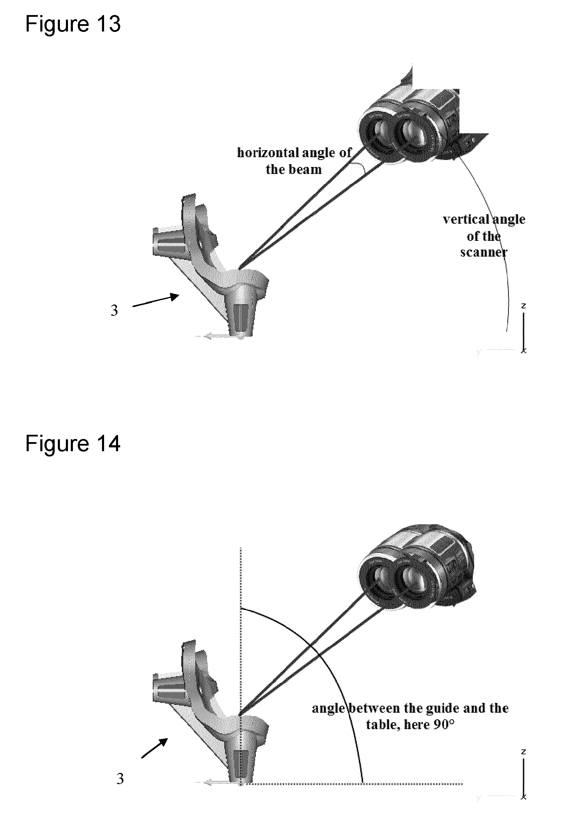

Figures 13 and 14 which generates the actually registered image according to a particular embodiment of the invention.

### DETAILED DESCRIPTION OF THE INVENTION

The current invention will be described on the basis of specific embodiments but is not limited to these embodiments; it is only limited by the scope of the claims. All references in the claims serve for illustrating purposes only and shall not be interpreted as limitations.

Any use of the term 'inclusive' in this application indicates that the possibility of additional steps or elements is not excluded. On the other hand, a description using the term 'inclusive' shall also encompass the embodiments that contain no other steps or elements than the ones summed up. Where an indefinite article is used to refer to a noun, it shall also include the plural form of the noun unless specifically stated otherwise.

The terms first, second, third, etc., if used in this application, only serve to distinguish similar steps and do not necessarily indicate the order of the elements or steps. Skilled persons will understand that under certain circumstances the order of elements and/or steps can change.

The terms 'operative use' and 'surgical' must be interpreted in a broad sense and encompass procedures within, as well as outside an operating theatre and include prosthetic, orthotic and orthodontic treatment. Consequently, a broad interpretation shall also be applied to the term 'pre-operative planning', which shall therefore include any planning involved in the production of a patient-specific medical device.

The different embodiments described in this application may be combined, even if that is not explicitly stated.

The terms or definitions provided in the application only serve to clarify the invention.

Surgical templates help the surgeon to accurately perform operations or surgical procedures by guiding the surgical instrument, e.g. a pin, drill, cutting or sawing instrument. More specifically, in patient-specific templates the surgical instrument is being guided according to a specific plan of the procedure performed on the patient. In this context, it is important to ensure that the template itself accurately determines the surgical actions that must be performed, in accordance with the patient's morphology and the surgical plan. Since the design and product process of the template contains several steps, this is not evident. Also, these methods involve unique surgical templates for specific procedures on specific patients.

The problem of quality control also presents itself in patient-specific implants. Deviations from functional parameters often are not discovered until the moment of placement, although even small deviations can have considerable impact.

According to a first aspect, the present invention provides optimised production methods for patient-specific medical devices and/or surgical equipment, more specifically (surgical or non-surgical) guides (or templates) and implants. The method enables obtaining certainty about the deviation of the produced product in one single action or step.

More specifically, the optimised methods for the production of patient-specific medical devices such as guides and implants according to the present invention include the step of directly comparing the geometry of the produced patient-specific device with the (pre-operative) planning data. The results of the comparison lead to either approval or disapproval of the produced instrument.

The optimised methods of the present invention thus check the critical functionality of the produced guide or implant. This not only provides much greater product reliability for the patient, it also ensures a more cost efficient production process compared to the current production processes, where for each instrument, the deviation in relation to the original design has to be checked after each intermediate step.

The methods according to this aspect of the invention are applicable to all patient-specific devices that require a planning, in other words where the (operative or non-operative) placement and/or the use of the device require that factors unrelated to the morphology of the device itself are taken into account, such as the anatomy of the environment of the placement, i.e. adjoining bone structures and tissue, more particularly blood vessels, nerves, muscle or fatty tissue, etc. In other words, this includes implants and any orthoses, as well as guides used for placement of implants and orthoses or for performing a surgical procedure (e.g. the correction of a bone fracture).

The creation of patient-specific implants and patient-specific templates or guides used for the placement of implants usually requires a planning. The design of a patient-specific implant often requires consideration not only of the morphology of the bone that needs to be replaced and the connection to the remaining bone structure, but also of how and where the implant will be attached. In patient-specific (surgical) templates, one starts from the location and orientation of the guide components for the surgical instruments, determined by the planning, and combines these with structural requirements that are defined, among other things, by the morphology of the bone and/or the implant to which the template must fit.

Production methods for patient-specific medical devices hence usually encompass the manufacture of the device on the basis of a design, the latter being made on the basis of (segmented) patient-specific images and planning data of the surgical procedure and patient-specific values derived thereof. More specifically, the manufacture of a patient-specific medical device is typically preceded by one of the following steps or a combination thereof:
1) collecting 3D patient-specific images
2) planning the surgical procedure based on the three-dimensional patient-specific images and the patient-specific values derived thereof, and
3) designing the patient-specific medical device with the use of the three-dimensional patient-specific images and the planning data.

Under certain circumstances, the planning and design steps can be carried out simultaneously. Planning is the centre point in the production of a patient-specific surgical template. For patient-specific implants, the planning can be integrated directly into the design.

The optimised production methods of the present invention provide for an additional step that performs a comparison between the values of one or more critical dimensions for one or more functional elements of the produced surgical template and the values of these critical dimensions as determined on the basis of the planning for these functional elements, whereby based on this comparison, the template is either approved or not. In particular embodiments absence of approval implies discarding of the device.

The optimised methods for the production of patient-specific surgical guides and implants according to the present invention use patient-specific (segmented) three-dimensional images of the anatomic zone in the patient's body where the procedure is going to be performed. The collection of three-dimensional patient-specific images is usually done by the practicing surgeon, dental specialist and/or (assisting) technical staff.

Methods for creating digital patient-specific images or image information are known to the skilled person and are generally described as 'Patient-specific Instrumentation Techniques'. These include, for instance, images made with a magnetic scanner (Nuclear Magnetic Resonance or NMR), a 'computer tomography (CT) scanner', a 'magnetic resonance imaging' or 'MRI' scanner or an 'ultrasound scanner'. The images taken of the patient are segmented and uploaded into a software programme that makes a detailed three-dimensional presentation of the tissue and bone of the patient. A summary of known medical image creating techniques can be found in 'Fundamentals of Medical Imaging', by P. Suetens, Cambridge University Press, 2002.

A next phase pre-plans the procedure based on the (segmented) patient-specific images of the anatomic regions that require treatment. For surgical templates, this includes, for instance, a description of the drilling routes, pin positions and saw cuts that are required for optimum placement of an implant.

More specifically, the methods according to the present invention plan the surgical procedure on the basis of the three-dimensional patient-specific images and the patient-specific values derived thereof. After all: the information concerning the location of the procedure, the specific requirements of the procedure and the surrounding tissue and/or bone structures that must be taken into account in performing the procedure, will differ for each patient. The planning of a patient-specific template or an implant will provide for a certain orientation, scope/form and depth with respect to the surgical procedure to be carried out, taking into account the specific values of the patient.

The planning of the action by the surgeon allows for exact determination of the location where surgical instruments must be used on the patient, as well as the desired shape, orientation and depth at which the instrument must operate. Factors such as the quality of the bone(s) and/or proximity or position of nerve bundles or blood vessels must be taken into consideration in this instance. This planning, combined with the patient-specific images, will then be incorporated into the design of the medical device (see below).

A planning may be carried out with adapted software programmes. It provides information about the functional elements that should be contained in the medical device to be used for the procedure, such as the guide or implant. The values of the critical dimensions of these functional elements can thus be established on the basis of the planning.

The planning is usually reviewed by the physician to ensure the correctness of the position and orientation of the functional elements.

The required functional elements of the medical device are laid down in the planning. In the context of the present invention, the term 'functional element' refers to an element of the medical device (e.g. the guide or implant) which ensures a certain functionality. Typical examples of functional elements in templates are the openings that allow penetration of one or more surgical instruments into the underlying bone or tissue, for instance a drilling cylinder, a drill hole, a cut or saw recess, a pin opening, etc. Typical functional elements in implants are screw holes. Furthermore, support surfaces and bonding characteristics also can be functional elements in both patient-specific devices. However, in the scope of the present invention, purely structural elements may, under circumstances, also serve as 'functional elements'.

Although most functional elements are determined directly by the planning, some elements may require post-processing action on the planning file. For instance: the planning of a knee implant will not directly describe the reference pin positions and pin orientations that must be included in the template. They can, however, be derived from the placement of the implant, based on algorithms known to the skilled person.

The functional elements are characterised by certain 'critical dimensions': parameters that are crucial for the operation of the functional elements and hence for the function and/or placement of the medical device. The critical dimensions usually refer to information on orientation (i.e. direction), form and/or dimension of the functional elements of the medical device. Usually they are also patient-specific.

Given the fact that these critical dimensions are determinant for the functionality and hence the usability of the medical device, they, or more in particular, their values as can be derived from the geometry of the produced medical device, compared to the values of the critical dimensions of the functional elements defined by the planning, can serve as the basis for the decision whether or not the medical device meets the requirements. Consequently, in most cases the critical dimensions of the functional elements can be derived directly from the planning data. Direct derivation of the critical dimensions may imply the necessity of performing an alignment. This is done via methods known to the skilled person, such as the classic methods (as described, for instance, by John Bosch, in 'Coordinate measuring machines and systems', Marcel Dekker Inc., 1995), or anatomic methods (as described, for instance, by Paul Besl in 'A method for Registration of 3-D Shapes, IEEE transactions on Pattern analysis and machine intelligence', vol.14, No.2 February 1992). Another option is to first convert the measured dimensions into a pseudo planning, or to re-calculate the planning into a set of dimensions.

The margins delimiting the guaranteed correct guidance of the surgical instrument through the surgical guide or the correct positioning of the implant, hence the correct and exact execution of the surgical procedure, can be checked for each of the critical dimensions. For instance, in the planning of a surgical procedure the value of the critical dimensions of the functional elements of a surgical template, e.g. the direction of a drilling cylinder, drill-hole or a cut or saw recess, will be determined as the value that is required to ensure correct guidance through the template of the drill or the blade of the surgical element. In certain embodiments, the critical dimensions also provide a minimum stable support surface on a bone or organ of the patient and the positioning of this surface in relation to the functional element of the patient-specific medical device. In this context it is important that the values are determined directly by the planning data rather than being based on information obtained in later production phases.

In the methods of the present invention, these values of critical dimensions as derived directly from the planning are compared to the values of these critical dimensions as determined by measurements of the produced (and either or not finished) instrument. Hence the values derived from the planning could be referred to as 'anticipated values' and those derived from the geometry of the produced product as 'actual values'.

In the creation of patient-specific medical devices, the planning phase is followed by a design or drawing of the patient-specific medical device on the basis of the preoperative planning, the three-dimensional patient-specific images and the patient-specific values derived thereof.

More specifically, a patient-specific device such as a surgical guide or implant is designed so as to integrate the functional elements of the medical device into a structure that matches the patient's anatomy. Based on the patient-specific images a patient-specific structure is provided to ensure a patient-specific match between the medical device and the patient's bone structure. This is usually realised by providing one or more patient-specific surfaces that are complementary to a bone-area of the patient.

In the design of guides, care is taken to integrate the functional elements into a structure that uniquely aligns with the bone, to ensure correct guidance of one or more surgical instruments after placement and stabilisation in this unique position. This implies the provision of, for instance, several specifically located openings with specific dimensions, depth and orientation that match the planning, and optionally of one or more support surfaces providing support to the functional elements on the one hand and ensuring that the placement of the guide or implant in the patient can only occur in the correct position, on the other.

Similarly, in the design of implants, the morphology of the implant that must align with existing bone structures will be equipped in the right places with the necessary functional elements, such as but not restricted to screw openings for attachment of the implant. As indicated above the functional elements can also consist of adjoining surfaces or supports.

Patient-specific guides and implants shall be designed such that after placement and stabilisation, they limit the surgeon's degrees of freedom to ensure that the actual procedure shows considerable concordance with the planning.

In particular embodiments, the patient-specific guides or implants comprise two or more parts, for example a femur guide and a tibia guide. In certain cases, each individual part may not be easy to identify on itself. Identification can be facilitated by coupling these parts to each other via a coupling element with a characteristic shape. Thus, in further embodiments, the design of the patient-specific device comprises the design of one or more coupling elements for coupling of the two or more parts of the patient-specific device, preferably in a locked relative position. In yet further embodiments, the design of the patient-specific device comprises the generation of a random shape for the coupling element(s). The (random) shape of the coupling element(s) makes it easier to identify the individual parts, and may be used as unique identifier for the patient-specific device. Furthermore, the coupling element may allow identification of the medical device when it is packaged, e.g. via x-rays.

The patient-specific medical devices are created on the basis of the design. Several techniques are available and known to the skilled person that can be used for the production of patient-specific surgical instruments. More specifically, guides or implants can be made by using 'additive manufacturing' techniques: the layer-by-layer or point-by-point application of a layer or specific quantity of material that subsequently is allowed time to cure. 'Additive manufacturing' techniques typically start from a digital three-dimensional presentation of the object to be produced (and in the context of this invention of the surgical guide or patient-specific implant to be produced). Generally, this digital presentation is subdivided into series of cross-sections of the object with the use of a computer system and 'computer-aided design and production' software that allows for digital stacking of the thus created layers in order to shape the object. The 'additive manufacturing' equipment subsequently uses this data for layer-by-layer creation of the real object.

The best-known 'additive manufacturing' technique is stereolithography (and related technology): selective layer-by-layer curing of, for instance, liquid synthetic material by means of a computer controlled electromagnetic beam.

Another 'additive manufacturing' technique is 'selective laser sintering', whereby powder particles are melted together according to a specific pattern and by means of an electromagnetic beam.

'Fused deposition modelling' is an 'additive manufacturing' technique whereby synthetic materials are brought together and stacked according to a specific line pattern.

'Laminated object manufacturing', on the other hand, is a technique whereby paper, plastic or metal plates are cut into a specific form with a blade and then glued together.

Finally, there is 'electron beam melting' technology: an 'additive manufacturing' technique that melts metal powder, one layer after another, by means of an electron beam and under vacuum conditions.

As indicated above and contrary to state of the art methods, the methods according to the present invention are characterised by the fact that (during and/or at the end of the production method) the produced patient-specific devices are compared directly with the original patient-specific planning data of the surgical procedure. More specifically, it involves a comparison in which the values of the critical dimensions for one or more functional elements that are present on the guide or the implant are compared directly with the values of the critical dimensions of the corresponding functional elements as provided in the original preoperative planning.

In other words, the methods according to the present invention check (one or more) critical dimensions for one or more functional elements of the guide or implant on the basis of the critical dimensions as derived directly from the planning data that also served as the foundation of the design.

The advantage of the methods according to the present invention is that comparisons and checks necessary to define and/or guarantee that within certain tolerance limits, the produced medical device meets the predefined standards of the functional elements of the device, require only one single step. The initial establishment of these standards in the preoperative planning and the direct comparison between the produced guide or implant and the planning data, minimises the potential risk of missing certain deviations in the finally produced device as compared to the standards laid down in the planning.

The methods of the present invention are also exceptional in that the produced guide or implant is not merely compared with the design (which may already contain errors), but that the functional elements are checked directly against the original preoperative planning.

The comparison step in the methods according to the present invention consists of a direct comparison between the values of one or more critical dimensions that have been determined from the measured geometry of the medical device and the values of the same one or more critical dimensions as derived directly from the preoperative planning data. In this context it is important that the values originate directly from the planning (and, optionally, from the original model of the bone structure) on the one hand and from the measurement of the produced device (either or not finished), on the other, hence that it does not involve values derived from steps following after the planning.

In certain specific embodiments of the methods according to this invention the comparison between the values of the critical dimensions of one or more functional elements as derived directly from the preoperative planning data and the values of the same critical dimensions as defined on the geometry of the medical device, is carried out at the end of the production process. The final product is thus linked directly to the original preoperatively planned critical values. However, it is not unthinkable that in addition to the comparison at the end of the production process, intermediate steps are also being checked in certain embodiments.

In certain embodiments, the comparison between values of the critical dimensions of one or more functional elements and the values of the same critical dimensions as derived directly from the preoperative planning data (either or not in combination with an additional comparison performed at the end of the production process), is carried out at an earlier stage of the production process. In some embodiments the methods according to the present invention can be combined with other measurements and checks.

In certain embodiments the step involving comparison of the values of one or more critical dimensions for one or more functional elements in the optimised production methods according to the invention comprises the following intermediate steps:
i) identifying one or more critical dimensions for one or more functional elements of the medical device
ii) defining the values of one or more critical dimensions identified in step i) by deriving these values directly from the preoperative planning data
iii) defining the values of one or more critical dimensions identified in step i) by deriving these values directly from the geometry of the produced patient-specific medical device; and
iv) making a comparison between the values of one or more critical dimensions as determined in step ii) and the values of one or more critical dimensions as determined in step iii).

Step (i) thus establishes, i.e. determines or identifies one or more critical dimensions for one or more functional elements of the produced surgical guide or implant. This implies establishing the functional elements as well as the critical dimensions for each of those functional elements. A next step ii) can then directly establish, calculate or derive the values of these critical dimensions based on the planning and the patient-specific images. As indicated above, this step can be carried out during the planning phase, or afterwards, on the basis of the planning data and the patient-specific images and/or values directly derived thereof.

The original planning that was typically made or approved by the surgeon and which, for a specific surgical procedure, reflects a certain orientation, scope/form and/or depth for the functional elements of the medical device, is stored in a computer system en can therefore in certain embodiments be used in step ii) as a basis for defining the values of one or more critical dimensions.

As indicated above, in certain cases, usually depending on the type of intervention, it may be necessary to perform a number of post-processing actions to the original planning in order to acquire the concrete values of certain critical dimensions. For instance, medical devices or objects that do not form part of the surgical guide or the implant - e.g. reference pins - usually will not be included in the original planning of certain surgical procedures. Their specific position and orientation hence not forming part of the planning, these will have to be derived from it or even calculated. It is important that this calculation is performed independently from the software and the operators used in the design of the guide or the implant in order to avoid interference with the data of the design. Failure to do so could mean that the comparison step in the methods of the invention does not take place only on the basis of the planning, and this could obstruct possible detection of certain deviations in the guide or the implant as compared to the planning.

As indicated above, the values of one or more dimensions of one or more functional elements that have been derived directly from the planning, are compared to the values of those critical dimensions as derived directly from the geometry of the produced (either or not finished) patient-specific medical device. Defining the values of these critical dimensions for the produced medical device can be done through measurement. An option is to optically scan the functional elements of a produced surgical guide or implant with the use of optical (or mechanical) scanning systems, such as a GOM scanner or other appropriate scanning device. Thus in particular embodiments the methods of the present invention comprises one or more measuring steps. In further particular embodiments the methods of the invention comprise the step of measuring the produced surgical guide or implant with the use of optical (or mechanical) scanning systems, such as a GOM scanner.

In certain embodiments, this determination is performed at the end of the production process, but as indicated above, the methods according to the present invention can also be carried out on the basis of measurements of the medical devices during the production process. In certain embodiments the measurements are carried out before the medical device is transferred to the final disinfection phase and/or sterilisation and packaging phase.

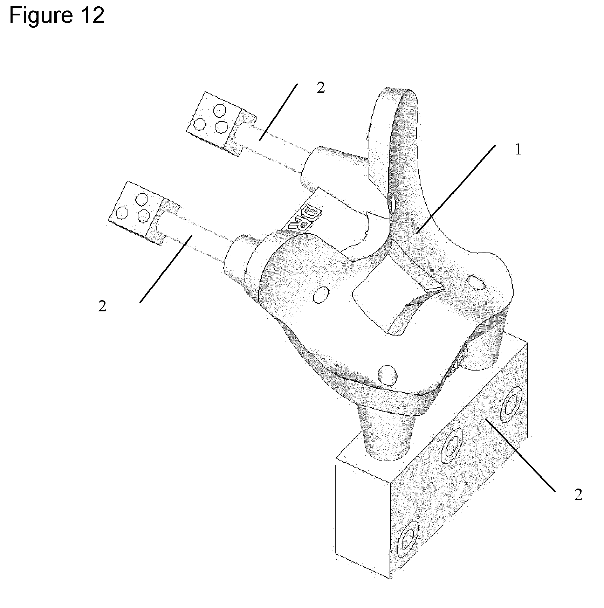

Depending on the critical dimensions that are to be scanned, calibrated references -e.g. calibrated reference blocks - can be attached to the produced surgical guide or implant and hence included in the measurement and/or scan. These may simplify the measurements of the guide or the implant (see Figure 12). Thus, in particular embodiments the methods of the invention comprise, in the generation of the device, the addition of one or more calibrated reference blocks attached to the surgical guide or implant. Such reference blocks can be produced simultaneously with the device or can be attached thereto after production of the medical device.

In certain embodiments of the method, the measurement values may be checked after the measurement procedure (e.g. after scanning). Such a check is useful to guarantee that the information is sufficiently detailed and accurate to describe the indicated geometry of the medical device within the measurement tolerances. The production methods according to the present invention thus encompass a comparison step in which the anticipated values of one or more critical dimensions (determined in the way as described in step (ii)) are compared to the 'actual' values of one or more critical dimensions (determined as described in step (iii)). In certain embodiments of the methods according to the invention, this comparison step (iv) involves the calculation of the deviation of the 'actual' values of the critical dimensions for the functional elements as determined on the guide or the implant, in relation to the 'anticipated' values for these critical dimensions based on the planning.

In certain embodiments, this comparison is carried out with an algorithm. Based on this calculation, it can then be determined whether or not the deviation falls within tolerable margins and whether or not the medical device meets the predefined requirements. In other words, the result of the comparison step in the methods according to the present invention forms a basis for either or not rejecting or disapproving the guide or the implant. In addition, it can also be used to inspect the reason of the deviation.

In certain embodiments of the methods according to the present invention, the step of comparing the values of one or more critical dimensions for one or more functional elements is preceded by establishing a unique link between the produced patient-specific medical device and the original (either or not segmented) patient-specific images.

This unique link enables unambiguous establishment of which patient-specific guide or implant matches which patient.

Linking the patient-specific images with the (data of the) guide or implant can be done on the basis of the inherent features of the guide or the implant itself, or by using an additional critical reference.

In certain embodiments an additional element is added to the produced medical device as an 'identification code' or label, which can take different shapes, e.g. that of an extrusion or protrusion, or that of a three-dimensional barcode that could be integrated into the surface of the guide or the implant, for instance. This allows for realisation of the critical reference through limited markings integrated into the scanned three-dimensional surfaces of the medical device. This type of code or label can also be applied to the three-dimensional surface of the project during the production phase. Additive Manufacturing (AM) makes this relatively easy: the label text can be incorporated into the geometry of the object by engraving the letters and/or digits into the design or adding them in relief and including them in the building process. This allows for easy identification of the object and for making a connection between the planning file and the patient.

Hence, the critical reference can be used to establish a connection, i.e. a unique link between the medical device and the patient.

In certain cases, engraved barcodes or barcodes which are added in relief can be difficult to be read by a scanner. Therefore, in particular embodiments, the barcode is provided on a separate part, which coupled to the medical device. In particular embodiments, the part comprising the barcode is clipped on the medical device. The separate part may be reusable and can be for example a small metal part. In particular embodiments, the barcode may be provided on the separate part by means of a sticker. In certain embodiments, the barcode is a Quick Response (QR) code, i.e. a two-dimensional barcode. In particular embodiments, the part comprising the barcode is removed from the medical device prior to packaging and shipping of the medical device.

In particular embodiments, the medical devices are provided with a radio frequency identification (RFID) tag. The RFID may be provided on a separate part, which coupled to the medical device.

In particular embodiments, the packaging provided for the medical device comprises a barcode. This barcode is then associated with the patient-specific medical device for which the packaging is provided. This ensures that the right medical device is placed in the right packaging, thereby ensuring that the medical device is shipped to the right customer.

In yet other embodiments of the present methods, the connection or unique link between the patient-specific medical device and the patient is made on the basis of inherent data of the produced guide, for instance the three-dimensional topology of the scanned surface of the guide or the implant. For example, one could use a critical dimension or a combination or set of critical dimensions as an 'identification characteristic'. In this context, for determination of the set it is important to take account of error tolerances that may occur in measurements of the patient-specific device. One must therefore choose the critical dimensions such that measurement errors cannot lead to a wrong identification. This requires considerable accuracy and measurement error analysis on the part of the skilled person who prepares the critical dimension set. It is also important that the uniqueness of the critical dimension or set of critical dimensions is checked on the basis of the planning.

On the other hand, the unique link between the patient-specific medical device and the patient can also be made by use of a statistical method, for instance the Principal Component Analysis (PCA) method, which allows for description of each patient-specific object through a unique combination of unique parameters. If these parameters can be derived from the scanned critical dimension(s), a unique link can be made between the measurement result and the planning file. In this embodiment, the uniqueness of the used identification can then be verified through statistical analysis and the link to the planning file subsequently made.

In this context, the present invention developed a method to ensure a unique link between a produced patient-specific medical device and the original segmented patient images. In the context of the production methods of the present invention, the unique link between the produced patient-specific medical device and the patient is thus established via a unique link between the original patient-specific images and the produced medical device. More specifically, a statistical method is used for the purpose, such as Principal Component Analysis. This method, which applies to the identification of each patient-specific device, constitutes another aspect of the present invention.

Accordingly, the present invention relates to (computer-implemented) methods for optimizing the production of patient-specific medical devices, which are characterised by at least the following steps:
a) determining the values of one or more critical dimensions for one or more functional elements as directly derived from the geometry of a patient-specific medical device;
b) determining the values of these critical dimensions as directly derived from the data of the preoperative planning for said patient-specific medical device;
c) comparing the values of the one or more critical dimensions for one or more functional elements as directly derived from the geometry of the patient-specific medical device and the values of these critical dimensions as directly derived from the data of the preoperative planning, as obtained in step a) and b) and
d) providing a signal for rejecting or accepting of the patient-specific medical device on the basis of the comparison carried out in step c. Whereby the provision of the signal is determined based on a threshold in the comparison step.

More particularly steps a and b are carried out based on the provided data of the geometry of the patient-specific medical device and of the preoperative planning which is carried out for the generation of said medical device.

More particularly, the invention provides computer programs which have the potential, to bring about when run on a computer, based on inputted data on the geometry of the patient-specific medical device and data of the preoperative planning to carry out steps a to d described above.

According to a second aspect, the present invention provides methods for the unique identification of a patient-specific medical device. This identification method is not limited to medical devices that require a planning, but instead applies to each patient-specific medical device, including patient-specific surgical guides or templates, orthoses or prostheses. Due to the use of inherent features of the medical devices, the use of a reference code or label may under certain circumstances become unnecessary.

In the production of large quantities of unique yet similar surgical guides, orthoses or prostheses, a simple method of identifying each produced object during or after its production is of great importance for several reasons. On the one hand, the often simultaneously produced objects need to find their way to the right client; on the other hand, during the different steps in the production process each additional action must be performed on the correct object. The traditional method of unique identification of produced guides, orthoses or prostheses is that of associating a unique label with each individual object. The label serves as the unique identification of the object. In its simplest form, this label could simply be a sequence number. More advanced known techniques are UUIs (Universally Unique Identifiers), often used in software for the identification of unique components. In the case of medical applications, the name of the patient can also be integrated in the label to enable direct human interpretation. A disadvantage of applied labels is that they change the geometry of the object by definition. In the case of applications for the creation of, for instance, medical devices such as guides, orthoses and prostheses, they often necessitate enlargement of the available surface on the object in order to place the label in a visible and non-functional area.

The identification methods according to the present invention allow for quick and easy assignment of the produced guide, orthosis or prosthesis to one well-defined set of patient-specific images and therefore also to the patient (and/or to the planning, if so desired). These methods apply to identification of each type of patient-specific device such as orthoses, prostheses (including implants) and guides or templates that are used for placing implants or for performing a specific procedure.

More specifically, the identification methods according to the present invention establish a unique link between the data of the produced patient-specific medical device and the original patient-specific images by using a statistical method, such as Principal Component Analysis.

The identification methods according to the present invention use the geometry of patient-specific medical devices such as surgical guides, orthoses or prostheses for identification. The geometry is usually unique due to the patient-specific characteristics and hence also linked to the patient involved. Furthermore, a non-functional physical adaptation of the object is usually not required.

However, the geometry of an object, for instance a medical device, is not easy to handle in itself and unpractical to serve as a key for data management. For that purpose, the geometry is efficiently encrypted through parameterisation of the optional geometries. In other words: the geometry of each possible variation of the object that is being produced can be described by a limited number of parameters, and vice versa. Each combination of parameters hence describes a possible geometry and fully defines it.

The indicated parameterisation of all possible geometries allows for statistical substantiation for which a large set of possible geometries is used as a reference dataset. A mean geometry can be calculated from this set.
The next step is to investigate where and to which extent the reference geometries vary as compared to this mean figure.

The different variation directions define the possible deviations in the geometry. The most important variation directions are called 'main variations'. The variation analysis can be carried out in different ways. The best-known method is the one that considers only linear variations and is referred to as Principal Component Analysis (PCA). A reproducing kernel can optionally be added to PCA in order to model nonlinearities. There are many other methods, which are often referred to as dimension reduction techniques (e.g. the ones described in Nonlinear Dimensionality Reduction, John A. Lee and Michel Verleysen, Springer, 2007). Each method has its merits and is best adapted in accordance with the available variations in the reference dataset.

If the reference dataset is representative of all possible variations that can occur for all objects, each new object can be described as a combination of these main variations. This means that the parameters of the combination fully define the geometry of each new object and hence are perfect candidates for the encryption.

A combination can be presented as a vector and it is easy to identify elements with a vector as a key.

If from a statistical viewpoint, the vector is undistinguishable from the existing vector, the (non-functional) geometry can be adapted, for instance by adding one or more details. The choice of the adaptation(s) must provide for a clear impact on the resulting vector. This method creates a unique geometry with a unique encryption.

In order to identify a produced patient-specific medical device, the object must be scanned. The scanning process produces a geometry and can therefore be described as a combination of the mean geometry and the main variations. The combination can be expressed as a vector. This vector is comparable with the vectors of objects that are already in the database.

The process of scanning and vectorising objects is subject to statistical variation. But since it involves vectors, it may be submitted to a simple statistical analysis and the equality (or almost-equality) of two vectors can occur with reliability intervals.

The identification methods according to the present invention can be applied in the optimised production methods pursuant to the first aspect of the present invention, which means that as a result of the efficient and unique link between the produced guide or the produced implant and the patient-specific images and their associated planning, the critical values of one or more critical dimensions as derived from the geometry of the guide or the implant can then be compared with the critical values of those one or more critical dimensions as derived from the original planning.

As described herein above, optical scanning may be used for assessing the geometry of the medical devices. In particular embodiments, the optical scanning is not only used for controlling the geometry, but also to identify the guide. This allows auto-sorting and quality control of all devices that pass the scanner. Uniqueness of data can be guaranteed by adding specific elements to the devices in the design phase.

Typically, in the context of the present invention, after production, the medical devices are packaged and shipped to the customer. More particularly, in the context of the present invention, if it is determined that the medical device meets the predefined requirements, the medical device is packaged and optionally shipped to the customer. However, this step generates an additional risk of error. In particular embodiments, an identification feature present on the device as described above may be used in the packaging and shipping steps, to identify the device. However, a further aspect of the invention provides that in the step of packaging particular features may also be introduced which allow identification of the device. In particular embodiments, the device or the packaging is provided with one or more fixtures which have a shape matching one or more (patient-specific) features of the medical device. This reduces the risk of shipping the wrong device to the customer. The one or more fixtures are typically designed based on the design of the medical device. In particular embodiments, the fixtures are manufactured using additive manufacturing. Where the medical device comprises two or more parts coupled via one or more coupling element as described herein above, the one or more fixtures may have a shape matching the shape of the coupling element(s). As indicated above, the coupling elements as such may also function as identification fixtures. Thus, the application further provides methods comprising the step of generating one or more fixtures based on the design of the medical device, packaging the device and the one or more fixtures and identifying the medical device in the packaging based on the specific features of the fixture. In further particular embodiments, the identification of the medical device based on the specific features of the fixture is performed by a scanning method.

A further aspect of the invention relates to computer-implemented methods for the unique identification of a produced patient-specific medical device with a patient, whereby a unique link is established between the geometrical data of the produced medical device and the original patient-specific images on the basis of a statistical method which includes the assignment of a unique combination of parameters to the geometry of the medical device. More particularly, the computer-implemented method comprises the steps of:
(i) providing a set of reference geometries of similar patient-specific medical devices
(ii) calculating a mean reference geometry based on the set of reference geometries
(iii) analysing the variation of the geometry of the patient-specific medical device as compared to the average reference geometry, and
(iv)assigning a unique combination of parameters that corresponds with the most explicit variations of the geometry of the particular patient-specific medical device relative to the average reference geometry.

More particularly, the invention provides computer programs which have the potential, to bring about when run on a computer, a unique combination of parameters that corresponds with the most explicit variations of the geometry of a particular patient-specific medical device relative to an average reference geometry, based on a set of reference geometries of similar patient-specific medical devices. More particularly, the set of reference geometries of similar patient-specific medical devices and the geometry of the patient-specific medical device is inputted into the program, whereafter the following steps are ensured:
(ii) calculating a mean reference geometry based on the set of reference geometries (iii)analysing the variation of the geometry of the patient-specific medical device as compared to the average reference geometry, and
(iv)assigning a unique combination of parameters that corresponds with the most explicit variations of the geometry of the particular patient-specific medical device relative to the average reference geometry.

In particular embodiments, the computer programs further have the potential, to bring about when run on a computer to obtain data from a scanning device in order to determine the set of reference geometries of similar patient-specific medical devices and the geometry of the patient-specific medical device.

The advantageous optimised production methods and identification methods according to the invention are further explained in the not-limiting description below and the appended figures.

### EXAMPLES

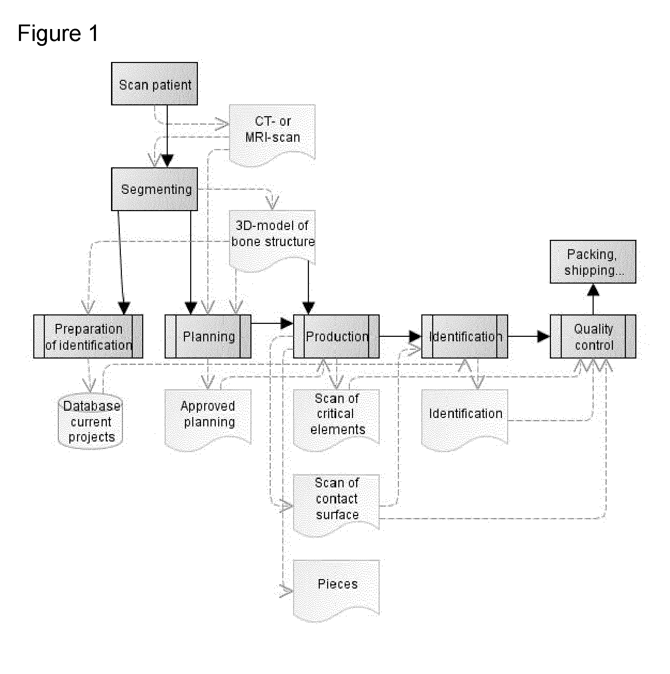

**Figure 1** provides an overview of a specific embodiment of the optimised production methods according to the invention, which relates to the production of a patient-specific surgical guide. Similar steps can, however, be described, for instance for a production process according to the present invention for patient-specific implants.

The overview in Figure 1 first shows a number of steps that precede the actual creation of the template, namely:
- collection of three-dimensional patient-specific images (scanning the patient and segmenting the images)
- planning the surgical procedure based on the three-dimensional patient-specific images (and the patient-specific values derived thereof). This includes the definition of, for instance, drilling routes, pin positions and saw cuts. And
- approval of the planning by the physician.

Subsequently, **Figure 1** describes the steps for creating the guide, namely:
- the design of the guide for support of the surgical procedure based on the data of the planning and the patient-specific images (and the patient-specific values derived thereof)
- production of the guide based on the design (followed by polishing and cleaning steps)
- the optical scanning procedure of the guide, which specifically includes scanning of the functional elements of the guide or the implant.

Characteristic of the methods according to the present invention is the quality check involving a comparison between the values of one or more critical dimensions as derived from the data of the preoperative planning on the one hand and the values of one or more critical dimensions as derived from the geometry of the produced patient-specific surgical guide on the basis of the optical scan, on the other.

Based on this comparison step the guide is then approved or rejected.

The embodiment of the method illustrated in Figure 1 also features an identification step (prior to the comparison step) whereby the data of the produced guide can be linked (i.e. identified) uniquely to the original patient-specific images.

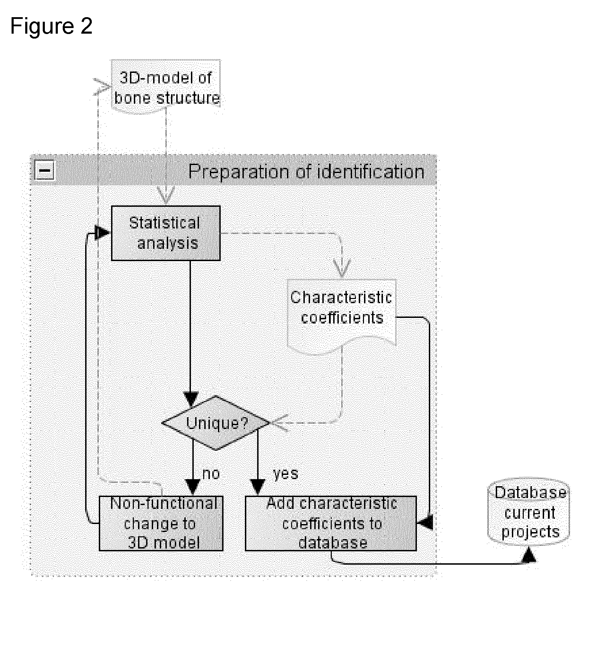

**Figure 2** gives a detailed overview of a specific embodiment of the identification step of the production methods according to the present invention, using a statistical method to ensure a unique link between the medical device and the original patient-specific images. Figure 2 shows that in the preparatory phase of this identification step, the use of a statistical method allows for describing the patient-specific images on the basis of a specific and unique combination of parameters (i.e. the characteristic coefficients). In these embodiments, the uniqueness of the used identification can then be certified through statistical analysis. In the event that it is not unique, a change to the bone surface is provided in the planning, for instance by adding a geometric element; in this context, attention must be paid to the fact that on the one hand, this should not affect the functional elements of the planning, but on the other hand, must produce a measurable deviation in the final medical device. In other words, a (non-functional) geometric element is added to the planning file on which the patient-specific medical device is based. This change ensures that the geometry is unique after all and included as such in the database.

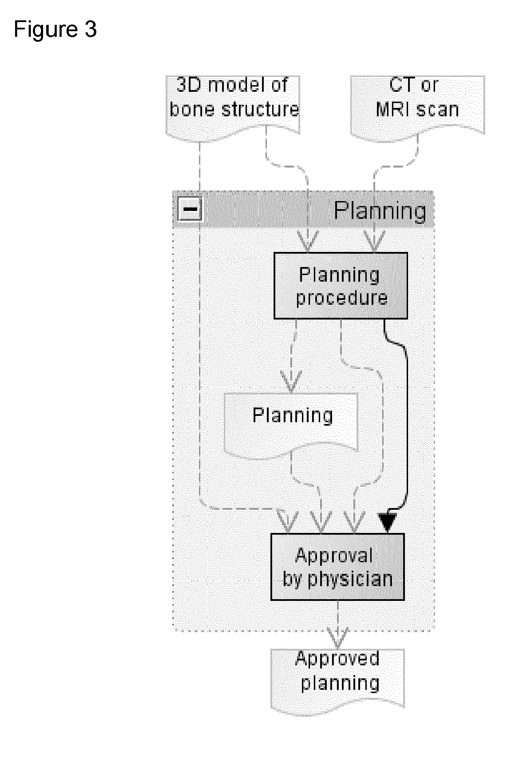

**Figure 3** shows how the preoperative step of the planning of a surgical procedure transpires according to the production methods of the present invention. In the production methods according to the present invention the surgical procedure is planned on the basis of the three-dimensional patient-specific images and the patient-specific values derived thereof. One thus obtains a simulation of the operation procedure whose output includes a planning with information on the functional elements that should be integrated in the guide or the implant. The critical dimensions of these functional elements can then be established on the basis of the planning. The planning is checked and approved by the (practicing) physician.

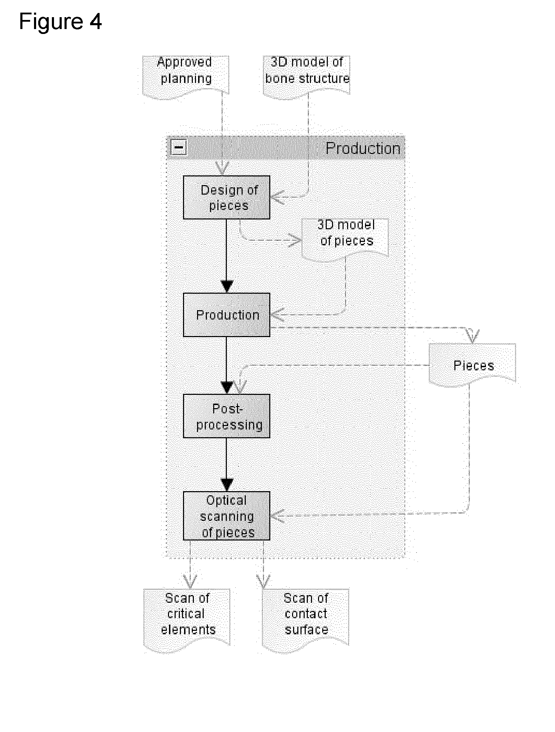

**Figure 4** gives a detailed overview of the different steps for the production of the patient-specific medical device, namely:
- the design of the medical device based on the data of the planning and the patient-specific images (and the patient-specific values derived thereof)
- the manufacture of the medical device based on the design (followed by polishing and cleaning steps), and
- the optical scanning procedure of the produced medical device, which specifically includes measuring the critical dimensions of functional elements of the medical device.

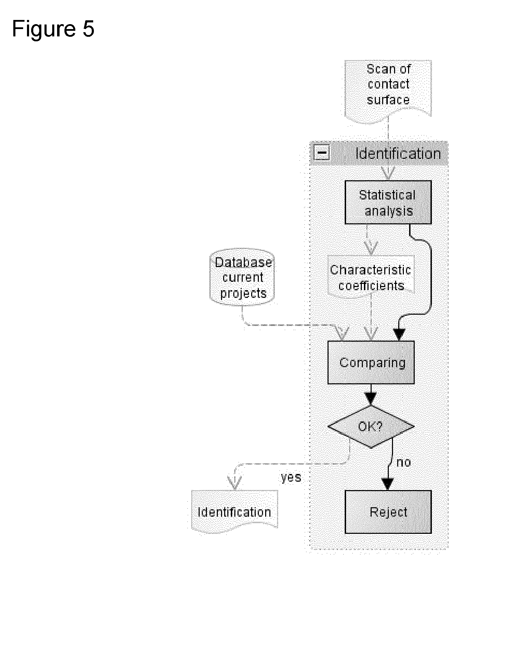

**Figure 5** shows the identification step according to certain embodiments of the production methods of the invention. Here, the unique link between the data of the medical device, such as the guide or the implant and the original patient-specific images, is realised by using a statistical method, for instance the principal component analysis (PCA) method, which enables description of each guide or implant through a combination of unique parameters (i.e. the characteristic coefficients). If these parameters can be derived from the scanned critical dimension(s) of the guide or the implant, the result of the measurement can be linked uniquely to the patient-specific images that with the use of the same statistical method, can also be described on the basis of the same combination of parameters. In these embodiments, the uniqueness of the used identification can then be certified through statistical analysis and the link to the planning file subsequently made.

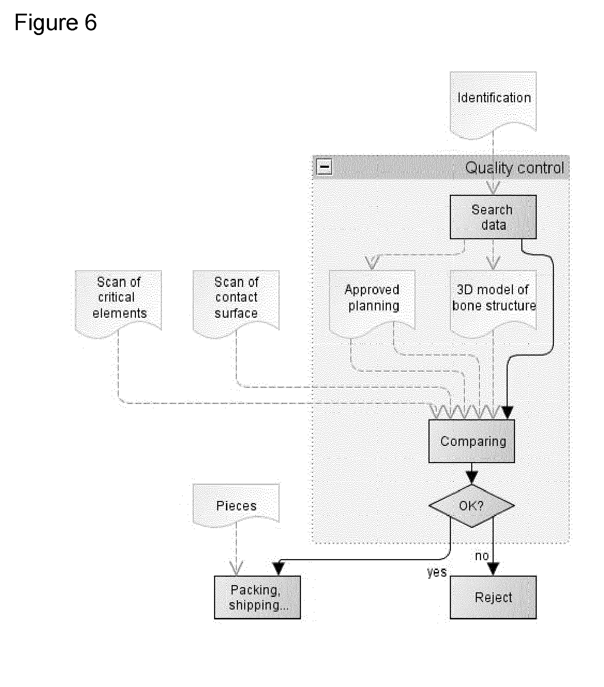

**Figure 6** details the quality check step of the production methods according to the invention. This quality check involves a comparison between the values of one or more critical dimensions as derived from the data of the preoperative planning on the one hand and the values of one or more critical dimensions as derived from the geometry of the produced patient-specific medical devices on the basis of the optical scan, on the other. Based on this comparison step the medical device is then approved or rejected.

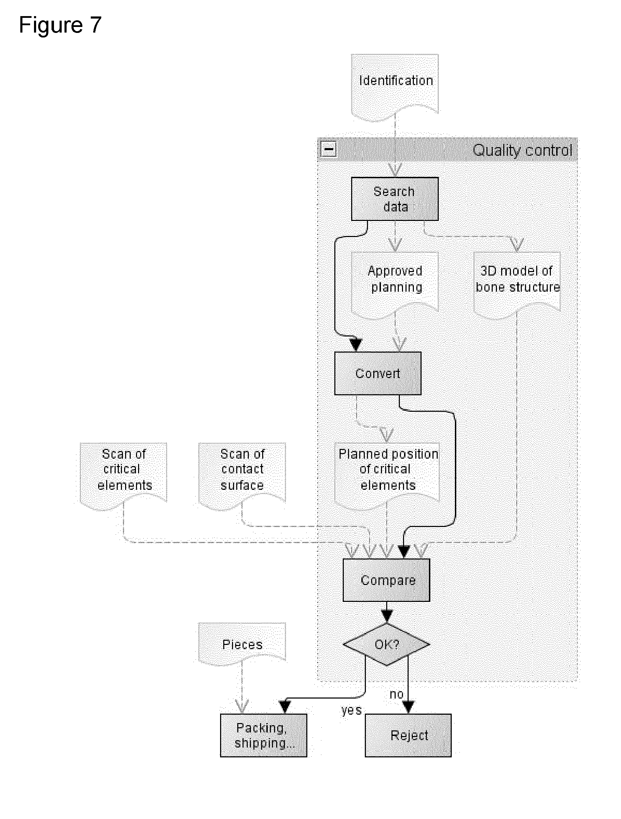

**Figure 7** In certain embodiments of the production methods according to the invention, as shown in Figure 7, the values of the critical dimensions are not directly indicated in the planning but must first be derived from it or even calculated. Hence it may occur that a number of post-processing actions must be performed on the planning data prior to arriving at the concrete values of the critical dimensions. Only then can the quality check step be carried out, i.e. the comparison between these values of the critical dimensions and the values of the critical dimensions as derived from the geometry of the guide or the implant.

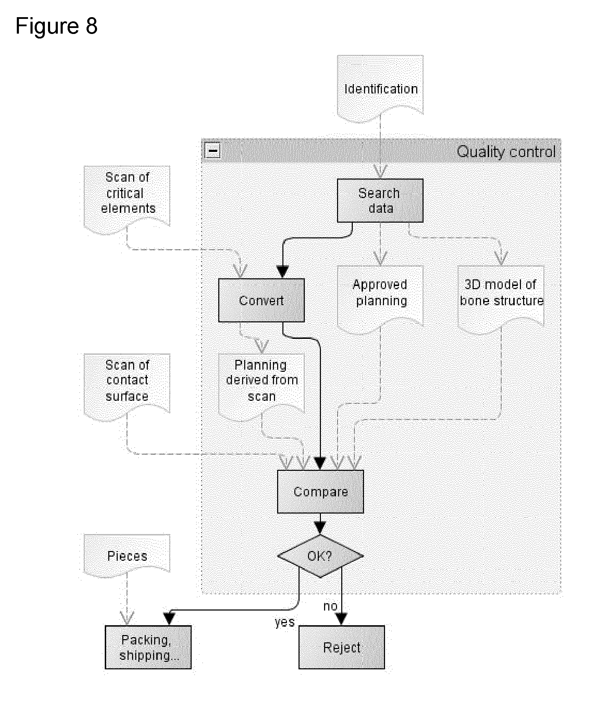

**Figure 8** In certain embodiments of the production methods according to the invention, as shown in Figure 8, prior to the quality check step of the optical scan of the produced guide or implant, a 'pseudo-planning' is derived that is subsequently compared to the planning approved by the physician.

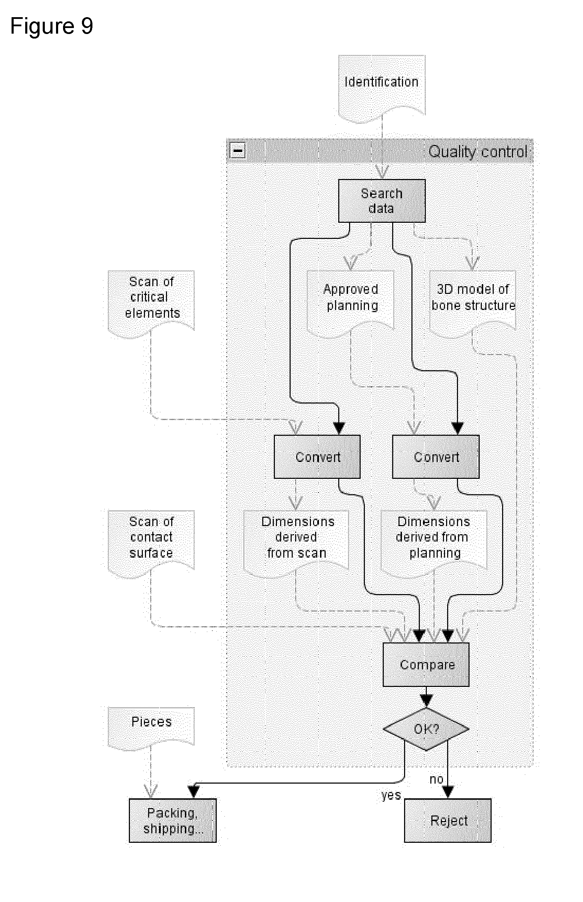

**Figure 9** In certain embodiments of the production methods according to the invention, as shown in Figure 9, the quality check step is preceded by a calculation of so-called derived dimensions on the basis of the values of the critical dimensions. This means that based on both the original planning and the optical scan of the produced guide or implant, the derived critical dimensions are calculated first and can then be compared with each other during the quality check.

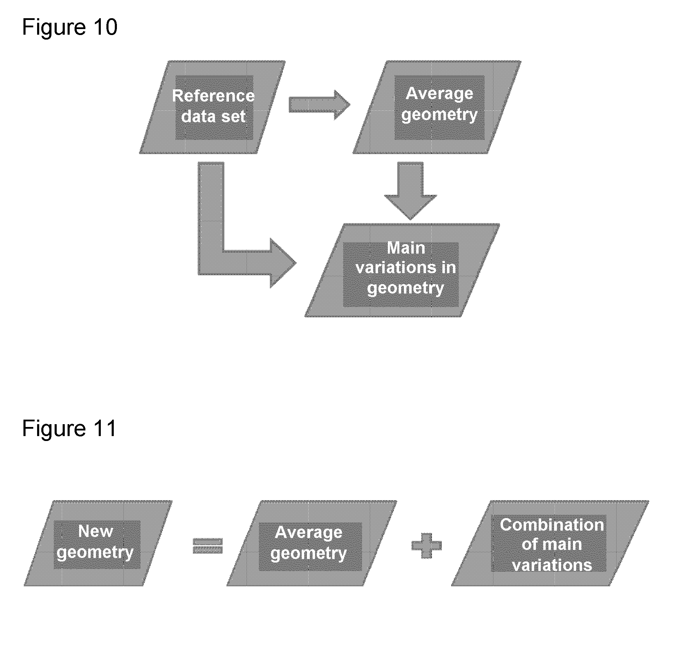

**Figures 10 and 11** reflect how the geometry of a certain guide or implant can be encrypted efficiently. According to the identification methods of the invention, this is done by describing the geometry of each possible variation of the object that is being produced by a limited number of parameters (i.e. the characteristic coefficients or variations) and vice versa. A large set of possible geometries is used as a reference dataset for this purpose. This set allows for calculation of a mean geometry. The next step is to investigate where and to which extent the reference geometries vary from this mean figure. The different variation directions define which deviations are possible in the geometry. The most important variation directions are called the main variations. If the reference dataset is representative of all possible variations that can occur for all objects, each new object can be described as a combination of these main variations. In order to identify a produced guide, orthosis or prosthesis, the object must be scanned. The scanning process produces a geometry and can therefore be described as a combination of the mean geometry and the main variations. The combination can be expressed as a vector. This vector can then be compared with the vectors of objects that are already in the database. The equality or inequality of two vectors, in other words, the uniqueness of the new geometry, can then be calculated according to statistical methods.

**Figure 12** According to certain embodiments of the production methods according to the invention, the values of one or more critical dimensions for one or more functional elements are defined on the basis of the produced surgical guide or implant by means of a measurement. In this context, the functional elements of the produced surgical guide or implant could, for instance, be scanned with an optical (or mechanical) scanning system. Depending on the critical dimensions that are to be scanned, calibrated references - e.g. calibrated reference blocks - can be attached to the produced surgical guide or implant and thus be included in the measurement and/or scan. This may simplify the measurements of the guide or the implant. Figure 12 shows a specific produced guide (1), equipped with calibrated reference blocks (2).

**Figures 13 to 15** show a schematic representation of an optical scanning procedure according to a particular embodiment of the present invention.

In particular embodiments, optical scanning of the medical device involves scanning the device with an optical scanner at a fixed set of angles. In particular embodiments, the set comprises five angles. This ensures that sufficient images of the medical device are taken, for example to calculate the critical dimensions of the device.

In particular embodiments, the medical device is placed on a table which is able to automatically rotate in a plane, for example a plane parallel to the floor. Typically, this is a 1-angle table, i.e. a table which rotates around a single rotational axis. The angular position of the table (and thus the medical device) with respect to a fixed reference is computer-controlled.

In particular embodiments, an optimized scanning procedure is followed. This procedure reduces the amount of angles, which enables a faster scanning process. Furthermore, the optimized procedure will, on average, increase the total coverage of each set of scans.

The first main goal of the optimized scanning procedure is finding the smallest possible set of angles needed to obtain the required information of the medical device.

Figures 13 and 14 shows a patient-specific medical device, more particularly a guide (3), which is positioned on a 1-angle table (not shown) in a certain position. The position of the guide relative to the table is fixed, but can be adjusted manually. This means that an optimal relative position (angle) can be fixed. Such an optimal position can be found using an algorithm (see further).

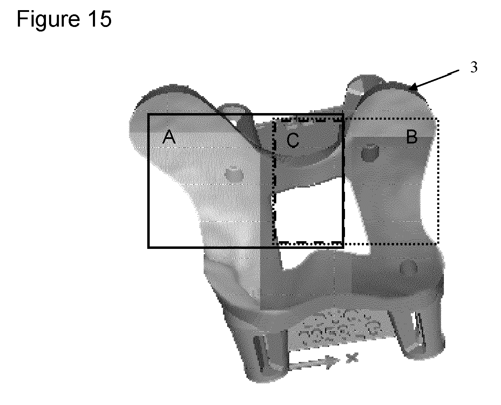

The scanner typically comprises a light emitting device which is positioned in between two cameras (or equivalent imaging means). Thus, the two cameras look at the guide from a slightly different angle. The intersection of the images taken by both cameras is the actually registered image. This is represented in Figure 15. If the first camera images area A (full lines) of guide (3) and the second camera images area B (dotted lines), the registered area of the guide is area C (dashed lines).

By rotating the guide around the Z-axis (perpendicular to the table), it is possible to cover more or another part of the guide surface. By applying multiple angles and by making the sum between the resulting covered surfaces (without counting overlaps between results twice), it is possible to determine if the guide surface is sufficiently covered by the registered images.

The algorithm which provides the optimal position or angle between the guide and the table, based on certain parameters, including:
- the vertical angle of the scanner, as shown in Figure 13
- horizontal angle between the beams, as shown in Figure 13
- input angle; i.e. angle between the table and the guide, more particularly the angle between the surface of the table and the Z-axis of the digital design file (STL file) of the guide
- minimum coverage required (as a percentage)

In particular embodiments, the output of the algorithm includes the following data:
- STL name (filename of the design file of the guide)
- Optimal angle between the guide and the table
- Amount of angles required using the optimal angle
- Set of imaging angles when using the optimal angle
- Amount of required angles using the input angle
- Set of imaging angles when using the input angle
- The resulting coverage (as a percentage) for every combination

The angles typically have an accuracy of 1°.

In certain embodiments, the table is a 2-angle table, i.e. a table which can rotate over two angles. This provides the ability to rotate the guide in any direction, without manual adjustment. In these embodiments, the output of the algorithm may further include the following data:
- Amount of required angles for a 2-angle table
- Set of angles for a 2-angle table.

The output parameters may be stored in an XML (Extensible Markup Language) file, which is then read by the scanner to rotate the table accordingly.

Thus, the algorithm solves the following scan optimization problems:
- Which is the smallest set of pair of angles that will result in a coverage of the guide surface higher than the specified minimum coverage?
- For a fixed vertical angle, which is the smallest possible set of horizontal angles that will result in a coverage of the guide surface higher than the specified minimum coverage?

A pair of angles is a pair (α, β) where α is the angle in the horizontal plane and β the angle in the vertical plane. For a 1-angle table, β may be fixed at the input parameter. Typically, the medical devices are oriented in such a way that the XY plane of the digital design file (STL file) coincides with the plane of the table. This means that the angle α (the angle in the horizontal plane) is the same as an angle in the XY plane or around the Z-axis, starting at the Y-axis. The β angle is applied around the X-axis and also starts from the Y-axis.

The α and β angles can be limited to angles between -90° and +90°. This means that there are 180² possible combinations of for α and β for a 2-angle table and 180 possible angles for the 1-angle table.

## Claims

1. An optimised method for the production of patient-specific medical devices, which method is **characterised by** at least the following steps:
1) collecting three-dimensional patient-specific images;
2) planning the surgical procedure based on said three-dimensional patient-specific images and the patient-specific values derived thereof;
3) designing the medical device based on the planning data, the patient-specific images and the patient-specific values derived thereof; and
4) manufacturing the medical device based on the design,
said method further comprising the steps of:
a) a comparison between the values of one or more critical dimensions for one or more functional elements as directly derived from the geometry of the patient-specific medical device and the values of these critical dimensions as directly derived from the data of the preoperative planning, and
b) the rejection or acceptance of the patient-specific medical device on the basis of the comparison carried out in step a.

2. The optimised method for the production of patient-specific medical devices according to claim 1, whereby the step of comparing the values of one or more critical dimensions for one or more functional elements encompasses at least the following intermediate steps:
i) identifying one or more critical dimensions for one or more functional elements of the patient-specific medical device
ii) defining the values of one or more critical dimensions identified in step i) by deriving these values directly from the preoperative planning data
iii) defining the values of one or more critical dimensions, identified in step i), by deriving these values directly from the geometry of the produced patient-specific medical device, and
iv) making a comparison between the values of one or more critical dimensions as determined in step ii) and the values of one or more critical dimensions as determined in step iii).

3. The optimised method for the production of patient-specific medical devices according to claims 1 or 2, whereby the step of comparing the values of one or more critical dimensions for one or more functional elements is performed at the end of the production process.

4. The optimised method for the production of patient-specific medical devices according to any one of claims 1 to 3, which comprises the step of optically measuring the produced patient-specific medical device to determination of the values of one or more critical dimensions as derived directly from the geometry of the produced patient-specific medical device.

5. The optimised method for the production of patient-specific medical devices according to any one of claims 1 to 4, whereby the step of comparing the values of one or more critical dimensions for one or more functional elements is preceded by establishing a unique link between the data of the produced guide or the produced patient-specific implant and the patient or the patient-specific images.

6. The optimised method for the production of patient-specific medical devices according to claim 5, whereby the produced surgical guide or the produced patient-specific implant contains a critical reference or a set of critical references and the unique link between the data of the produced patient-specific medical device and the patient or the original set of patient-specific images is established on the basis of this critical reference or set of critical references.

7. The optimised method for the production of patient-specific surgical guides and implants according to claim 6, which comprises providing an identification code on said medical device.

8. The optimised method for the production of patient-specific surgical guides and implants according to claim 7, whereby the identification code is integrated three-dimensionally into the surface of the patient-specific medical device.

9. The optimised method for the production of patient-specific medical devices according to claim 8, whereby a unique link of the data of the produced patient-specific medical device is made with the patient-specific images based on the geometry of the medical device including said identification code.

10. A computer-implemented method for optimizing the production of a patient-specific medical device, which is **characterised by** at least the following steps:
a) determining the values of one or more critical dimensions for one or more functional elements as directly derived from data of the geometry of said patient-specific medical device;
b) determining the values of these critical dimensions as directly derived from data of the preoperative planning for said medical device;
c) comparing the values of the one or more critical dimensions for one or more functional elements as directly derived from the geometry of the patient-specific medical device and the values of these critical dimensions as directly derived from the data of the preoperative planning, as obtained in step a) and b); and
d) providing a signal for rejecting or accepting of the patient-specific medical device on the basis of the comparison carried out in step c.

11. A computer program which has the potential, to bring about when run on a computer, based on inputted data on the geometry of a patient-specific medical device and data of the preoperative planning to carry out the following steps:
a) determining the values of one or more critical dimensions for one or more functional elements as directly derived from data of the geometry of said patient-specific medical device;
b) determining the values of these critical dimensions as directly derived from data of the preoperative planning for said medical device;
c) comparing the values of the one or more critical dimensions for one or more functional elements as directly derived from the geometry of the patient-specific medical device and the values of these critical dimensions as directly derived from the data of the preoperative planning, as obtained in step a) and b); and
d) providing a signal for rejecting or accepting of the patient-specific medical device on the basis of the comparison carried out in step c.

12. A method for the unique identification of a produced patient-specific medical device with a patient, whereby a unique link is established between the geometrical data of the produced medical device and the original patient-specific images on the basis of a statistical method which includes the assignment of a unique combination of parameters to the geometry of the medical device.

13. The identification method according to claim 12, whereby at least the following steps are carried out for the assignment of a unique combination of parameters to the geometry of a certain patient-specific medical device:
(i) providing a set of reference geometries of similar patient-specific medical devices
(ii) calculating a mean reference geometry based on the set of reference geometries
(iii) analysing the variation of the geometry of the patient-specific medical device as compared to the average reference geometry, and
(iv)assigning a unique combination of parameters that corresponds with the most explicit variations of the geometry of the particular patient-specific medical device relative to the average reference geometry.

14. The identification method according to claim 13, whereby step (iii) involving the analysis of the variation of the medical device geometry, relative to the average reference geometry, is carried out through principal component analysis.

15. The identification method according to claims 12 to 14, whereby, in the event that explicit variations could not be established in the critical dimensions of the planning, or, consequently, in the ensuing geometry of the particular patient-specific medical device, and a (non-functional) geometrical element is added to the planning file that forms the basis of the patient-specific medical device.
